(19) Europäisches Patentamt
European Patent Office
Office européen des brevets

(11) EP 2 845 624 B1

(12) **EUROPEAN PATENT SPECIFICATION**

(45) Date of publication and mention
of the grant of the patent:
**07.06.2017 Bulletin 2017/23**

(51) Int Cl.:
*A61P 17/00* (2006.01)   *A61K 36/185* (2006.01)
*A61K 47/06* (2006.01)

(21) Application number: **14184267.4**

(22) Date of filing: **10.09.2014**

(54) **Mucoadhesive devil's claw extracts (harpagophytum procumbens) and uses thereof**

Mukoadhesive Teufelskrallenextrakte (Harpagophytum procumbens) und deren Verwendung

Des extraits de griffe du diable (harpagophytum procumbens) mucoadhesives et leur utilisation.

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2013 IT RM20130501**

(43) Date of publication of application:
**11.03.2015 Bulletin 2015/11**

(73) Proprietor: **Aboca S.p.A. Società Agricola
52037 Sansepolcro (AR) (IT)**

(72) Inventors:
• **Mercati, Valentino
52037 Sansepolcro AR (IT)**
• **Mattoli, Luisa
52037 Sansepolcro AR (IT)**

(74) Representative: **Predazzi, Valentina et al
Società Italiana Brevetti S.p.A.
Piazza di Pietra, 39
00186 Roma (IT)**

(56) References cited:
**EP-A1- 2 397 136      WO-A1-2010/037213
DE-B3- 10 326 556      DE-B3-102005 020 330
FR-A1- 2 915 900       US-B1- 6 280 737**

• **ABDELOUAHAB NASSIMA ET AL: "Dermal and transcutaneous delivery of the major glycoside constituents of Harpagophytum procumbens (Devil's Claw) in vitro", PLANTA MEDICA, vol. 74, no. 5, April 2008 (2008-04), pages 527-531, XP002723039, ISSN: 0032-0943**
• **GRANT L ET AL: "A review of the biological and potential therapeutic actions of Harpagophytum procumbens.", PHYTOTHERAPY RESEARCH : PTR MAR 2007, vol. 21, no. 3, March 2007 (2007-03), pages 199-209, XP002723038, ISSN: 0951-418X**

Remarks:
The file contains technical information submitted after the application was filed and not included in this specification

## Description

The present invention relates to a fraction of a devil's claw extract *(Harpagophytum procumbens)* in which low concentrations of iridoid glycosides (in particular harpagoside, to which all devil's claw-based preparations are standardized) and resins are present, a process for the preparation of said fraction of extract, compositions comprising it, and the use of the fraction of extract and of the compositions for the protection, or to cooperate with the protection of skin or mucous membranes or as carriers with mucoadhesive properties.

PRIOR ART

Devil's claw is the name generally given to desert plant *Harpagophytum procumbens,* usually found in the South and East of Africa, in savannah land and Kalahari desert areas.

Devil's claw is a plant whose root has typically been used to treat fevers, rheumatoid arthritis, tendinitises, contusions, back aches, osteoarthritis, sciatica and the like, and has a content of iridoid glycosides, measured as harpagoside content, higher than about 2.5%.

Moreover, other iridoid glycosides such as harpagide, procumbide, procumboside, are present.

Moreover, devil's claw contains phenolic substances such as verbascoside, flavonoids, triterpenes, sugars, resins, minerals.

Iridoid glycosides, present in devil's claw, are the active principles which give to the latter antirheumatic and antiinflammatory properties comparable to those of synthetic anti-inflammatory drugs. Harpagoside is reported to be in general about 80% of devil's claw iridoids, therefore it is used as reference to standardize devil's claw-based products.

The root extract proved to have, thanks to iridoid glycosides, analgesic, anti-oxidizing, digestive properties.

Commonly, the dry extract of devil's claw contains 1 to 3% of harpagoside, even though dry extracts containing up to 8% of harpagoside can be found, and the various known extraction methods are focused on improving the yield in these components or on making such components more useful for therapeutic purposes.

In the literature it is reported how mucoadhesive materials can be useful for therapeutic purposes or to carry drugs, or as therapeutic agents as such, coating and protecting damaged tissues (for instance, gastric ulcers or lesions of the mucous membranes) (J.D. Smart 2005 Advanced drug delivery reviews 57 pp 1556-1568).

Given the medical interest for mucoadhesive substances and for those of natural origin, it is useful to develop novel natural mucoadhesive compounds to be used as drug carriers or for a therapeutic/protective aim.

SUMMARY OF THE INVENTION

The Authors of the present invention have identified mucoadhesive substances of natural origin which are advantageously depleted in active principles which would not enable their general use as drug carriers or as agents for prevention/therapy with a mechanical protective effect of damaged or irritated mucosal membrane coating. The present invention relates to a fraction of a devil's claw extract substantially depleted in the known pharmacologically active components represented by iridoid glycosides titrated in the component most represented, i.e. harpagoside, of which only traces remain, and to a process for obtaining such fraction. The fraction according to the invention may be used for a purpose completely different from that for which the devil's claw extract is used in the state of the art; said use could be for the protection or to cooperate with the protection of the mucous membranes or the skin, and/or also to retain desired substances on mucous membranes thanks to the mucoadhesive abilities of the selected fraction. The fraction of the invention therefore provides a new source of material of plant origin useful for the protection of the mucous membranes and of the skin, and/or also as pharmaceutical or parapharmaceutical carrier capable of retaining for a longer time desired substances on the mucous membranes, wherein the pharmacological effects attributable to the components known as active principles are substantially reduced or even eliminated. Said condition is particularly desirable when materials of natural or plant origin are to be used for actions of a mainly mechanical type (i.e., not for pharmacological actions having, for instance, a direct effect on the activation of receptorial, immune pathways or metabolic processes). The use of substances having a mainly mechanical effect, such as, e.g., substances having a barrier or mucoadhesive effect, is desirable, for instance, to cooperate with a pharmacological therapy. By minimizing the presence of substances with pharmacological activity in the material of plant or natural origin as in present invention, it is obtained a material with a mainly mechanical effect that can be used in combination with drugs, minimizing possible undesired interactions with drugs, at the same time exploiting the typical feature of substances of plant origin, of having a vast number of components capable of exerting a protective effect besides the pharmacologically active ones commonly used.

The use of fractions of extracts of plant or natural complex substances depleted in pharmacologically active principles entails the dual advantage of providing a material with a mainly mechanical protective action (mucous membrane protection and/or skin protection) and of enabling a concrete and useful use of materials normally discarded in extraction processes of plant or natural active principles.

The present invention therefore relates to a fraction of a devil's claw extract depleted in iridoid glycosides wherein the glycoside harpagoside has a concentration in weight percent at least of ≤ 1.3% and a weight percent of soluble macromolecules with a molecular weight of >25000 Da of >4%, its use to cooperate with the protection of skin or mucous membranes and/or as carrier with a high mucoadhesive ability; compositions comprising said fraction; their use in the protection of skin or mucous membranes and/or for the carrying of desired active principles on the mucous membranes and processes for the preparation of such fraction and of such compositions.

The invention also relates to a process for the preparation of a fraction of devil's claw extract as defined above, comprising the following steps:

a. preparing a hydroalcoholic extract of devil's claw roots, said extract is subjected to centrifugation and/or decantation, obtaining a clarified alcoholic extract;
b. obtaining from the clarified alcoholic extract prepared in a. a concentrated aqueous fraction;
c. decanting and/or centrifuging and filtering said aqueous fraction, collecting a water-soluble fraction C characterised by a concentration of harpagoside, expressed as weight percent, of ≤1.3%.

The invention also relates to the preparation of mixtures of said fraction C with one or more natural extracts and/or active principles deriving from sources different from devil's claw, such mixtures, as well as the preparation of pharmaceutical compositions or of medical foods containing said fraction in combination with other natural substances and/or active principles deriving from sources different from devil's claw and the use of such fraction alone or mixed with other natural components, to cooperate with the protection of the mucous membranes and of the skin or as carrier capable of retaining desired active substances (deriving from sources different from devil's claw) on the mucous membranes by virtue of the effective mucoadhesive properties of said fraction.

The invention therefore relates to a fraction of a devil's claw extract and its uses for the protection of mucous membranes or of skin or to cooperate with said protection, or as carrier with good mucoadhesive properties wherein said fraction is depleted of at least 50% of iridoid glycosides and is therefore markedly reduced in the effects attributable to commonly used active principles of devil's claw.

DETAILED DESCRIPTION OF THE FIGURES

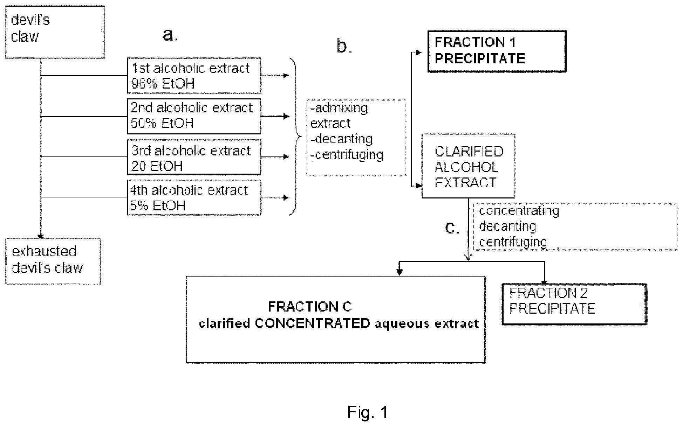

Figure 1 shows a block diagram of an embodiment of the process of the invention.

SHORT GLOSSARY

FILTRATE: fraction that has passed through the clarification filter

By "not higher", with respect to the amounts of active principles in fraction C, in the present description it is meant ≤.

By "iridoid glycosides", in the present invention are meant total iridoid glycosides titrated in harpagoside. For the purposes of the present description, the concentration of harpagoside present in the reference devil's claw extract or in the fraction of the invention is the concentration expressed as weight percent with respect to the weight of the lyophilized extract or fraction.

DETAILED DESCRIPTION OF THE INVENTION

As indicated above, a fraction of a devil's claw extract depleted in iridoid glycosides titrated as harpagoside is provided, wherein said harpagoside has a concentration in weight percent of ≤ 1.3%. By applying the extraction process object of the invention, described below, it was possible to obtain such fraction of devil's claw depleted in resins and in iridoid glycosides, which concomitantly surprisingly exhibits a greater protective action on skin and on mucous membranes with respect to the non-fractionated devil's claw extract, as evident from the mucoadhesivity assays reported in the experimental section below.

With respect to the dry extracts and/or lyophilized extracts of devil's claw and to the devil's claw root as such, which comprise an amount of iridoid glycosides of at least about 2.5-3% in weight, the fraction of extract of the present invention has merely traces of such active principles, as comprising an amount of harpagoside at least of ≤1.3% in weight.

Evidently, such percents correspond in practice to a very strong reduction in iridoid glycosides, and the pharmacological effects of such principles will therefore be strongly reduced.

Since the process as described leads to the concentration of resins in a well-defined fraction that does not match the selected fraction C, the mucoadhesive effect of fraction C of the extract is not ascribable to resins.

The fraction selected according to the present description, substantially depleted in active principles as the iridoid glycosides titrated in their major representative, harpagoside, exhibits however improved mucoadhesive properties with respect to the (non-fractionated) reference extract, as can be seen in Table 1 below.

The Table also shows the mucoadhesive properties of a lyophilized hydroalcoholic extract definable as total Devil's claw extract, used as reference

Table 1.

| Fraction | Mucoadhesion on inclined plane % |
|---|---|
| Reference Devil's claw extract, lyophilized | 47% |
| Hydrosoluble Devil's claw Fraction C, lyophilized | 84% |

The depletion in Devil's claw most known active principles, represented by iridoid glycosides, is directly measurable by harpagoside measuring.

Harpagoside measuring as reported in the present description is obtainable with standard processes by HPLC chromatographic determination.

The analysis method provides sample extraction with 80% methanol by ultrasounds at a temperature of 35°C $\pm$3°C, and HPLC analysis by c8 reverse-phase column and UV detector at a wavelength of 278 nm.

The mucoadhesive properties of the fractions are evaluated by a cell adhesion assay, it also described below. Evidently, such properties can also be evaluated with further techniques known to a person skilled in the art.

The fraction C according to the invention has a concentration of iridoid glycosides titrated in harpagoside of $\leq$1.3%, therefore a depletion in iridoid glycosides of about 50% with respect to the starting extract; a composition of hydrosoluble macromolecules with a molecular weight of >25000 Da, equal to about 4.2%, with respect to the 3.0% of the starting extract, and a mucoadhesion ability, calculated by adhesion assay with mucin on an inclined plane described below, equal to about 84%. Therefore, fraction C is depleted of about the 50% in iridoid glycosides with respect to the reference (or starting) devil's claw extract consisting of the extract lyophilizate in 70% alcohol, and shows a >40% increase in mucoadhesive abilities with respect to the starting hydroalcoholic extract which could be, for instance, the hydroalcoholic extract as described herein.

Therefore, the fraction of extract of the invention has the concentrations of iridoid glycosides titrated in harpagoside and hydrosoluble macromolecules with a molecular weight of > 25000 Da, indicated above and in Table 2, which can also be defined.

In one embodiment, the fraction of the invention has a concentration of iridoid glycosides titrated in harpagoside equal to about 50% with respect to a devil's claw root lyophilizate in 70% alcohol (e.g., ethanol), a concentration of hydrosoluble macromolecules with a molecular weight of >25000 Da equal to about 130% with respect to a lyophilizate of devil's claw root extract in 70% alcohol (e.g., ethanol) and a mucoadhesive ability measured by mucoadhesion assay on inclined plane higher than 150% (or even higher than 170%, equal to about 178%) with respect to a lyophilizate of devil's claw root extract in 70% alcohol (e.g., ethanol).

In other terms, the fraction of the invention is depleted in active principles represented by iridoid glycosides titrated in total harpagoside of about 50%, is enriched in soluble macromolecules having a molecular weight of >25000 Da of about 25% and has an increase in mucoadhesive abilities higher than 40%.

The mucoadhesion percentage of the fraction of the invention was calculated by adhesion assay with mucin on an inclined plane reported below and is equal to about 84%.

The mucoadhesion assay on an inclined plane is normally carried out on an inclined plane coated with a mucin film. The percentage (percent) of mucoadhesion is calculated as % difference = (adhered mucin %- adhered blank %)/ adhered % of the blank.

The adhesion assay on an inclined plane is known in the literature and is briefly summarized herein even though, as evident, the mucoadhesion percent could be measured according to any method known to a technician in the field. In particular, the mucoadhesion percentage can be measured according to a simple mucoadhesion assay on an inclined plane that is explained in a detailed manner in the experimental section and which, in short, consists in measuring the mucoadhesive ability of a sample by measuring its ability to bind to mucin disposed on an inclined plane, allowing the moving away by sliding of the sample lacking mucoadhesive ability.

The method provides the use of an inclined plane which acts as support for the biological substrate, consisting of a mucin film set up by depositing, on a plexiglas plane kept horizontal, a suspension of gastric mucin (e.g., porcine) at a certain concentration in a buffer with physiological pH.

The plane is dried to obtain a film of known surface area.

An amount of known weight of the sample to be analyzed is prepared and deposited on the measurement plane with a multichannel pipette at constant time and rate for some seconds.

The total amount is measured at each assay.

The sample which does not stop on the plane is collected and measured at the end of the run. The amount of fallen sample is measured by recording the weight variation as a function of time. As control, measurements in the absence of the mucin film on the inclined plane are also performed (measurements indicated as blank) with the same amounts of sample and under the same testing conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

$$\% \text{ difference} = (\text{adhered mucin \%} - \text{adhered blank \%})/ \text{ adhered \% of the blank}$$

Where:

- adhered mucin % = sample percentage remained adhered to the mucin film at the end of the measurement
- adhered blank % = sample percentage at the end of the blank measurements.

The fraction of the invention, though being depleted in harpagosides, is instead enriched in soluble macromolecules with a molecular weight of > 25000 Da. With respect to a conventional devil's claw extract, wherein the percentage of soluble macromolecules with a molecular weight of > 25000 Da is of about 3%, in the fraction of the invention such percentage is increased to over 4%.

Hereinafter, technical data related to the fraction of the invention and the starting extract and other fractions of extract obtained with different extraction steps are provided.

The table below shows how the mucoadhesivity of the various fractions of devil's claw analyzed be not ascribable with certainty on the basis of the weight percent of soluble macromolecules with a molecular weight of > 25000 Da or on the basis of the weight percent of harpagoside.

Fraction A corresponds to the precipitate obtained from extraction in 96% ethanol.
Fraction B corresponds to the precipitate obtained from extraction in 15%-5% ethanol.
Fraction C corresponds to the fraction obtained with the process of the invention.
Fraction D corresponds to the adsorbed fraction obtained by subjecting the water-soluble fraction obtained in c. to a step on high-porosity adsorbing styrene and DVB copolymer resin.
Fraction E corresponds to the retentate fraction obtained by subjecting the water-soluble fraction obtained in c. to a step on filter with a cutoff of 1000 Da.
Fraction F corresponds to the eluted fraction obtained by subjecting the water-soluble fraction obtained in c. to a step on filter with a cutoff of 1000 Da.
Fraction G corresponds to the retentate fraction obtained by subjecting the water-soluble fraction obtained in c. to a step on filter with a cutoff of 2500 Da.
Fraction H corresponds to the eluted fraction obtained by subjecting the water-soluble fraction obtained in c. to a step on filter with a cutoff of 2500 Da.

Table 2.

| SAMPLE DESCRIPTION | Hydrosoluble macromolecules with MW>25000>Da (%) | harpagoside (%) | Mucoadhesion on inclined plane |
|---|---|---|---|
| REFERENCE DEVIL'S CLAW EXTRACT, LYOPHILIZED | 3.08 | 2.65 | 47% |
| DEVIL'S CLAW Fraction A | 2.28 | 0.42 | 11% |
| DEVIL'S CLAW Fraction B Precipitate - 15%-5% etOH extraction | 2.53 | 9.13 | 23% |
| DEVIL'S CLAW Fraction C | **4.20** | **1.29** | **84%** |
| DEVIL'S CLAW Fraction D | 5.32 | 18.,57 | 38% |
| DEVIL'S CLAW Fraction E | 3.67 | 2.60 | 28% |
| DEVIL'S CLAW Fraction F | 13.84 | 5.39 | 101% |
| DEVIL'S CLAW Fraction G | 3.44 | 2.53 | 31% |
| DEVIL'S CLAW Fraction H | 9.3 | 4.20 | **70%** |

The table above shows that the fraction of the invention is the best with respect to the starting extract and to other analyzed fractions obtained from the reference extract in terms of mucoadhesivity with respect to the depletion in harpagoside.

The Inventors of the present invention have selected the above-mentioned fraction with respect to other fractions obtainable with the process as described in the present document as it was the only one exhibiting sufficient reductions in iridoid glycosides and maintaining, or better exhibiting, an increase in mucoadhesive abilities.

Many of the fractions obtained, in fact, besides exhibiting an enrichment in active principles, and therefore having a marked pharmacological effect, in some cases exhibited no mucoadhesive activity.

Therefore, evidently it is difficult to foresee which fractions of a plant extract be capable of exhibiting a mucoadhesion effect.

The fraction of the invention, represented by fraction C as defined above and obtainable by the above-described process, is therefore useful for its mucoadhesion properties in all those cases in which a protection of skin or mucous membranes is needed or desirable, which may be cases wherein a drug which attacks the mucous membranes or the skin has to be administered, therefore in order to prevent or limit the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes undergo irritations or alterations whereby the presence of mucoadhesive properties can prevent or limit damages on the skin or the mucous membrane. The mucoadhesive properties of the selected fraction in fact enable a direct protection of the mucous membrane or the skin, and also enable to use such fraction as carrier with mucoadhesive properties, or a carrier enabling a more effective presence of the active principles mixed therewith on the mucous membranes, thereby increasing the effectiveness itself of the desired pharmacological active principles.

In particular, the carrier could be a pharmaceutical or parapharmaceutical carrier comprising or consisting in the fraction of extract according to the invention, characterized by promoting the adhesion of active principle to mucous membranes.

By indicating the effect of protection of the mucous membranes, or of cooperating with the protection of the mucous membranes, it is meant a mechanical protection, given by the mucoadhesive abilities of the fraction itself, or also a pharmacological protection related to active principles that are preferentially carried to the mucous membranes when mixed to the fraction of the invention thanks to the mucoadhesive abilities thereof. In the present description it is understood that said mucous membranes can be the oral mucosa, gastric mucosa, intestinal mucosa, the nasal mucosa, the vaginal mucosa, the uterine mucosa, the rectal mucosa.

Fraction C could be used alone or could be mixed with extracts of other plants and/or active principles of other plants in order to boost the mechanical activity, as defined above, of the resulting mixtures.

According to the invention, the fraction could be obtained by the process described hereinafter.

Process for the preparation of a fraction of a devil's claw extract depleted in iridoid glycosides titrated in harpagoside, comprising the following steps:

> a. preparing a hydroalcoholic extract of devil's claw roots, subjecting said extract to centrifuging and/or decanting, obtaining a clarified alcoholic extract;
> b. obtaining from the clarified alcoholic extract prepared in a. a concentrated aqueous fraction;
> c. decanting and/or centrifuging and filtering said aqueous fraction, collecting a water-soluble fraction C with a concentration of harpagoside, expressed as weight percent, of $\leq$1.3% and a concentration of soluble macromolecules having a molecular weight of >25000 Da equal to $\geq$4%, and collecting said fraction C.

The fraction C obtainable by the above-described step c. exhibits very interesting features reported in Tables 1 and 2 above, i.e.:

> About 50% depletion in active principles, mucoadhesion percentage by adhesion assay, measured by adhesion assay with mucin on an inclined plane reported below, equal to about 84%, about 25% enrichment in soluble macromolecules having a molecular weight of >25000 Da.

The extract obtained according to the process of the invention will have a concentration of harpagoside, in weight percent, of $\leq$1.3%, and high mucoadhesive abilities.

The process described herein could be carried out starting from dry or lyophilized devil's claw root extracts, restoring such dry extracts in hydroalcoholic solutions containing about 70% alcohol in a 1:10 ratio.

According to the invention, the above-described process can be carried out by performing at step a. at least two, at least three, or at least four steps in ethanol at decreasing concentrations, wherein the decreasing concentrations of ethanol is from about 96% ethanol to about 5% ethanol.

For instance, step a. of the process can be carried out by performing a step in (with) about 96% ethanol, and a step in about 5% ethanol, between which a step in about 50% ethanol and/or a step in about 20% ethanol can optionally be performed.

Therefore, step a. of the process could be carried out by performing a step in about 96% ethanol, a step in about 50% ethanol, a step in about 20% ethanol and a step in about 5% ethanol.

The extracts obtained as described above are gathered to form a hydroalcoholic extract. These can be gathered, e.g., in equal mutual ratios, such as 50:50 in case of two steps in ethanol, or such as 33.3:33.3:33.3 in case of three steps in ethanol or 25:25:25:25 in case of four steps in ethanol. Evidently, the extracts obtained by performing the various steps in ethanol described above could be gathered in to different ratios, according to the general knowledge of the technician in the field.

In a preferred embodiment, step a. of the process is carried out by performing a first step with (in) about 96% ethanol, a step with (in) ethanol at a volume comprised between about 15% and 5% to concentration starting from the residue obtained in the preceding step, then mixing the two extracts 1:1 to have an extract with a resulting ethanol volume comprised between about 45-50%.

In any case, the extraction process, and therefore also step a. according to the invention, is carried out at temperatures which do not exceed 50°, in particular the step in 96% ethanol can be performed at about 40°C and the next step in 15-5% ethanol is performed at temperatures which do not exceed 50°C.

In the process according to the invention, when desiring to deplete the fractions from harpagosides rather than enriching them, the high temperatures generally used in processes in which the making of harpagosides-rich fractions of devil's claw is sought are not necessary.

As already indicated above, the hydroalcoholic extract obtained in step a. will be used to prepare a concentrated aqueous fraction at step b.

In c., the aqueous fraction is decanted and/or centrifuged so as to form a precipitate and a supernatant, thereby constituting the concentrated aqueous fraction that will be further filtered to obtain fraction C.

Decantation could be performed, e.g., 1 to 72 hours, and could be followed or replaced by one or more centrifugations to about 3000-4000 (e.g., about 3500) rpm for a period comprised between 1 and 10 minutes, e.g. about 5 minutes.

The supernatant obtained at step a. is then collected and subjected in b. to alcohol evaporation by standard techniques, like, e.g., by use of a thin film distillation system, or a batch concentration system according to standard protocols commonly used by the technician in the field. At this point, according to the invention, an aqueous fraction is then prepared, concentrated 5 to 10 times with respect to the starting solution in order to facilitate separation of the insoluble fraction between 45-50% ethanol and water, which precipitates by alcohol evaporation.

The result of this evaporation is a concentrated aqueous fraction of extract that is further purified by filtration to obtain fraction C in step c.

The concentrated aqueous fraction (which could also be referred to as concentrated aqueous solution) may be subjected to decantation for a period comprised between 1 and 10 hours, and the supernatant is then recovered, thereby obtaining a clarified solution.

The concentrated aqueous fraction, further decanted or not decanted, is filtered, thereby obtaining a clarified solution which corresponds to fraction C of the invention. In some cases it will be possible to perform one or more successive filtrations of the extract on filters having gradually decreasing cutoffs, from 200 micrometers down to 0.1 micrometers. The filtration could be performed, e.g., on a panel filter filtering with a cutoff of 25 micrometers.

The filtrate obtained as described above is then recovered as fraction C and has the features described above.

The fraction obtainable with the process of the invention is depleted in iridoid glycosides and comprises harpagoside at a concentration in weight (percent) of ≤1.3%.

The process according to the present invention, wherein the order of steps as described above therefore enables to obtain a fraction of a devil's claw extract as described above, which is depleted in iridoid glycosides and surprisingly rich in substances having a mucoadhesive action. The above-described process also eliminates resins via the reagents and steps used, as evident to the technician in the field.

Therefore, the fraction of the invention will also be substantially free from resins that will be found in the discarded fractions.

In a specific embodiment, the process of the invention can be summarized as follows:

Step a. extraction at about 40°C with 96% ethanol + extraction at about 50°C with 15-5% ethanol > 1:1 mixing of the two extracts.

Step b. obtainment of a concentrated aqueous fraction from the extract prepared in a., subjecting the alcoholic extract purified at about 45%-50% obtained in a. to alcohol evaporation, concentrating it about 5- to 10-fold, and

Step c. precipitate separation > obtainment of the fraction enriched in protective substances.

The present invention also relates to a composition comprising the fraction in any one of the above-described embodiments, useful in the protection or in cooperating with the protection of skin or mucous membranes.

Said protection, of course with reference both to the fraction per se and to the composition, could be a preventive protection or a protection of partially compromised skin or mucous membranes.

The present invention also relates to a natural carrier having the above-described mucoadhesive abilities, which consists in or comprises the above-described fraction C.

The skin lesions according to the present invention are, e.g., lesions that may involve also tissue underlying the skin and in which no open wounds are present, or in which open wounds are present.

By skin lesions not involving the presence of open wounds, according to the present description, are meant those lesions in which the superficial layer of the skin, and the underlying layers, though not being wounded, are particularly fragile, irritated and damaged.

Non-limiting examples of this type of lesions are represented by first-degree burns, first-degree decubitus lesions, pressure lesions, newly-cicatrized rashes, wounds or burns, irritations, erhytemas.

The composition or the fraction of the invention could then be used to cooperate, in association with suitable active substances (not extracted and therefore not deriving from Devil's claw), with the treatment of skin and/or mucous membranes for the prevention or the slowing down of worsenings of the same thanks to the good mucoadhesion and cell adhesion (assayed by other assay) abilities of the fraction described herein.

The compositions according to the invention could advantageously comprise further components, e.g. of plant origin, having, e.g., emollient, digestive, prokinetics, cholagogue, carminative, prebiotic, relaxing, excipient, preservative, humectant properties.

When the composition or the fraction of the invention are used for the protection or to cooperate with the protection of the skin, the application thereof will be topical. The embodiments of the compositions for topical use are reported hereinafter in the description.

The composition according to the invention could be made, e.g., as oil-in-water emulsion, water-in-oil, oil-in-gel or gel-in-oil emulsion, multiple emulsions, sprays, and anhydrous formulations (pomade, gel, paste, cream, ointment) and will comprise one or more excipients suitable for the making of the desired end form.

Such excipients could be, e.g., besides fraction C itself that, as described herein, can be used as mucoadhesive carrier, emulsifying agents (cetearyl alcohol, cetearyl glucoside, hydrogenated castor oil), rheological additives, antioxidants (like, e.g., vitamins, tocopherols or other antioxidants known in the field).

The emulsifying agent could be a surfactant, which by lowering the interfacial tension decreases the free energy

EP 2 845 624 B1

of the system; alternatively, also non-surfactant substances can be used, such as gum arabic, gelatin, hydrophilic colloids or finely subdivided powders (e.g., talc). In one embodiment, the excipient could be present at an overall concentration in weight comprised between 3 and 8%, such concentration being of course indicative, taking into account that anyhow the technician in the field will know how to adapt the concentration of the necessary excipients according to the embodiment he/she intends to prepare without addition of inventive activity.

Moreover, the composition could comprise perfuming and/or coloring agents making its odor pleasant, like, e.g., one or more essential oils like, e.g., lavender essential oil, melaleuca, lemon, mint, orange essential oil, and/or coloring agents enabling, e.g., to easily recognize the zones of application of the composition, the coloring being, e.g., temporary so as not to interfere with other, subsequent applications of the composition.

In one embodiment, said coloring and/or perfuming agents are comprised at an overall concentration in weight between 0.001 and 3%.

By "overall concentration in weight" it is meant the concentration in weight in the composition of the sum of the various excipients, or the concentration in weight in the composition of the sum of the various perfuming and/or coloring agents present in the composition.

As to the use of the composition in the protection of the skin or to cooperate with the protection of the skin, the invention also relates to medical devices like, e.g., medicated plasters, medicated gauzes, medicated bandages, medicated tissues, medicated tampons, medicated diapers (pads), i.e. plasters, gauzes, bandages, tissues, tampons or diapers (pads) which comprise, or be at least partly covered by, or be at least partly soaked with the described composition of the invention in the most appropriate form.

The gauzes could be made as fatty gauzes, and so the bandages and the plasters, by using the composition made in the form, e.g., of ointment, paste or cream. The tissues could be soaked with the composition in the form of emulsion of oil with water or gel, and the diapers (pads) or the tampons could be made by inserting the composition in the suitable layers known in the field for the insertion of protective or anti-irritating compositions.

Such products, like, e.g., diapers, tampons (commonly called "sanitary napkins") for women and pads for adult incontinence, are commonly used, e.g., in the babyhood/childhood-related field, in the feminine field and in the geriatric field, and the technician in the field will know where to insert the composition described herein, and which embodiment thereof be the most suitable one, with no need of specific teachings and by basing him/herself solely on techniques conventional in the field.

Such devices will be applicable over the part to be treated and/or protected by way of prevention.

As already indicated above, the composition of the invention may be a composition for topical use which could be realized in the form of oil-in-water emulsion, water-in-oil emulsion, multiple emulsions, spray, and anhydrous formulations (pomade, ointment, gel, paste, cream, spray,) according to techniques commonly used in the field. The formulation indicated herein as "spray" could be an anhydrous formulation or even an emulsion formulation, the form with an atomizer enabling also self-applications in zones harder to reach (like, e.g., the back), useful in all those cases in which the formulation is used, e.g., to alleviate sun rashes, erythemas, or skin irritations of various kind.

According to another embodiment, the fraction or the composition of the invention could be used, thanks to their mucoadhesive effect, for the protection o to cooperate with the protection of the mucous membranes.

In this case as well, such protection could be a preventive protection, e.g. in all those cases envisaging an administration of drugs having the side effect of attacking the mucous membranes, or in those patients exhibiting conditions of recurring irritation of the same. In other cases, the protection could be instead a protection for curative purposes or in order to avoid the worsening of irritated or partially compromised mucous membranes.

In these cases, the administration of the fractions or of the composition could be topical for all those mucous membranes on which a topical administration is possible (e.g., oral, rectal, vaginal, nasal mucosa) or oral in the cases in which said membranes be, e.g., intestinal or gastric mucous membranes.

The compositions for the protection of the mucous membranes could therefore be made in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

In case of formulations for topical use, the above indications related to the compositions for the protection of the skin could be followed, or syrups, rinses, sprays could be made, e.g. for the protection of the mucous membranes of the mouth, throat, nose.

For application on the rectal mucosa, suppositories or enemas or microenemas could be used, with the excipients known to the technician in the field for the making of such of compositions.

As already mentioned, the composition of the invention could be in the form of capsule, tablet, lozenge, hard gelatine, soft gelatine, granule, powder, syrup, elixir, suspension, emulsion. For oral administration the composition could be made in the form of daily unit dosages or of fractions of daily unit dosages (e.g. 2, 3, 4, 5, 6, or more capsules, tablets, lozenges, granule or powder single-doses, or gelatins could be taken over the day, according to the judgment of the doctor in charge), and may contain conventional excipients, including, e.g., binding agents, like gum arabic, gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, e.g. potato starch; and moisturizers like sodium laurylsulphate. The tablets can be coated according to methods well-known in the standard pharmaceutical practice.

The composition could also be made in a liquid or semiliquid form, as a suspension, emulsion, solution for oral administration, and could optionally contain natural aromatizing agents giving a palatable taste thereto.

The composition in the form of powder or granule could be pre-metered in suitable containers and ready for use, either by ingestion as such or to be resuspended in an appropriate liquid such as water, tea, etc. In this case as well, the composition could contain natural aromatizing agents giving a palatable taste thereto.

As already mentioned, the composition could comprise the fraction of the invention as carrier with a strong mucoadhesive activity in association with suitable drugs, or could instead comprise the fraction of the invention for a sole mechanical-type protection. In all cases, the composition could comprise further excipients as indicated above.

Evidently, all of the above-indicated excipients could be used in a pharmaceutically acceptable grade.

In one embodiment, the composition as described herein, in any one of the above-indicated embodiments, could be in the form of pharmaceutical composition, i.e. comprise pharmaceutical-grade ingredients, or it could be or be introduced into a special-purpose food or into a medical device.

The composition according to the present description could be made in the form of pharmaceutical composition or of medical device according to any one of the classes described in Directive 93/42 EEC on medical devices (comprising also substances and not only "devices" in the mechanical sense of the term), or in the form of medical food, food supplement or in any form according to the regulatory provisions of the Country in which said composition will be produced.

The medical device or the medical food could also contain as ingredients other components comprising, e.g., combinations of vitamins, mineral salts and other substances aimed at diet supplementing.

The fraction or the composition of the invention are therefore useful for their mucoadhesion properties as carrier or as main component in all those cases in which the protection of skin or mucous membranes is needed or desirable, which may be cases wherein a drug which attacks the mucous membranes or the skin has to be administered, therefore in order to prevent or limit the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes undergo irritations or alterations whereby a barrier effect can prevent or limit damages on the skin or the mucous membrane.

The invention also relates to a process for the treatment or for the prevention of the onset or the worsening of skin lesions, wherein such process comprises one or more applications of the composition of the invention or of the medical device comprising it once or more per day on the concerned part.

The application of the composition, for instance, could be repeated whenever needed (e.g. at each change of pad in case of prevention of incontinence-related rashes) or once, twice, thrice, four or more times per day in general.

The invention also relates to a process for the preparation of a composition according to any one of the embodiments described above, wherein a fraction of a devil's claw extract depleted in iridoid glycosides, wherein said iridoid glycosides titrated in harpagosides have concentration in weight percent of ≤1.3, is mixed with at least one of an excipient and/or substances of natural and/or plant origin having emollient, digestive, prokinetics, cholagogue, carminative, prebiotic, relaxing, excipient, preservative, humectant properties as described above, wherein said substances are not active principles extracted from devil's claw.

Among these, e.g., could be used one or more of: gentian root extract, boldo leaves extract, milk thistle fruits extract, artichoke leaves extract, taraxacum root extract, anise (or aniseed) fruit extract, rosemary leaves extract, mint leaves extract, marjoram leaves extract, cumin fruit extract, coriander fruit extract, ginger root extract, fennel fruit extract, caraway fruit extract, althea root extract, arnica flower extract, jojoba oil, charcoal, inulin, aloe gel. Object of the present invention is also a process for the protective (preventive or curative) treatment of skin or mucous membranes, providing the administration of the fraction or the composition of the present invention to a patient in need thereof.

Therefore, by "protective treatment" for the purposes of present invention, it is meant a treatment of damaged, irritated or irritable (e.g., if the use of an irritating drug is envisaged) skin or mucous membranes wherein the fraction or the composition of the invention form a protective film thanks to the mucoadhesive power of the fraction of the invention, and thereby enable a healing of the damaged, wounded and/or irritated mucous membranes, or prevent damage thereto which may be caused by therapeutic treatments that must be administered to the patient, by administering the fraction or composition of the invention before the drug.

Alternatively, the fraction or composition could be administered together with active principles that is desirable to retain longer on the mucous membrane in order to prolong or make more effective the therapeutic effect thereof.

Such administration could also be concomitantly with the administration of other drugs.

The strong reduction in pharmacologically active devil's claw principles in the fraction or in the composition of the invention makes such products particularly suitable to administration concomitantly with other drugs, as a side effect of interaction between the fractions, or between the composition of the invention and the drug coadministered or concomitantly administered is markedly unlikely.

A non-limiting example of the process of treatment and/or of prevention of the skin or the mucous membranes, could comprise the administration of a daily dosage, subdivided into a single dose or plural doses, of the fraction or the composition according to the present description, for a period of time comprised between one and six weeks, e.g. comprised between three and six weeks, or even for a period of time higher than six weeks, according to the judgment of the doctor in charge.

Such administration could precede the administration of the drug even for a prolonged period, so as to optimize the health state of the skin or of the mucous membrane to be treated.

The doctor in charge will know how to establish both the most appropriate dosage and the administration times, also on the basis of the patient's health state, weight, gender and age.

One of the substantial differences between the structure of the skin and that of the mucous membranes is represented by the absence, in the mucous membranes, of a selective barrier such as the corneous layer. Oral mucous membranes contact with noxious or irritating substances present in the environment (pollutants, pathogenic microorganisms, etc.) can therefore cause a high penetration of said substances both inside the mucous membranes and in the related airways (bronchi, lungs, etc.) causing inflammatory and/or allergic pathologies.

The protective effect of the fraction of the present invention was assessed by mucoadhesion assays which aim at evaluating the mucoadhesivity of the product under examination in order to establish whether such product has the ability to adhere to the mucous membranes and consequently exert a protective action.

For instance, a simple design is available for evaluating mucoadhesivity of the product of interest, which assesses the mucoadhesive ability of the sample by measuring its ability to bind to mucin disposed on an inclined plane, allowing the moving away by sliding of the sample lacking mucoadhesive ability.

**Evaluation of adhesion percentage by inclined plane with mucin.**

Mucoadhesive ability measurements are performed by an equipment comprised of a 45°-inclined plane, thermostated to 37°C; below said plane a microbalance is set, which performs measurements at regular 1-s intervals and prints recorded measurements on paper. Performed measurements are then transcribed and reprocessed on Excel spreadsheet.

The inclined plane acts as support for the biological substrate, consisting of a mucin film set up by depositing, on a plexiglas plane kept horizontal, a volume equal to 3ml of a suspension of porcine gastric mucin at an 8% concentration (w/w) in a phosphate buffer, pH 6.4.

The dispersion is then brought to dryness at the temperature of 44°C so as to obtain a film of known surface area equal to 33.6 cm$^2$.

An exactly weighed amount of the fraction according to the invention is dissolved in a solvent. The fraction is then deposited on the measurement plane with a multichannel pipette at constant time and rate during 6 seconds.

The multichannel pipette is calibrated so as to meter the solution (or dispersion) over the work plane. The total amount is measured at each assay (the weight of said volume is measured before the assay and depends on the density on the density of the sample under analysis).

The sample, which will fall on the microbalance at the end of the run, is loaded in the top part of the inclined plane, containing the mucin film, and let slide thereon. The amount of fallen sample is measured by recording the weight variation as a function of time. The assay as made herein lasts 40 seconds, though usually after 20 seconds there is no weighing variation.

Measurements in the absence of the mucin film on the inclined plane are also performed (measurements indicated as blank) with the same amount of sample and under the same testing conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

% difference = (adhered mucin % - adhered blank %)/adhered % of the blank

Where:

- adhered mucin %= sample percentage remained adhered to the mucin film at the end of the measurement
- adhered blank % = sample percentage at the inclined plane at the end of the blank measurements.

EXAMPLES

**Example 1**

**Preparation of the fraction according to the invention**

Devil's claw roots were fragmented and subjected to two steps of extraction in ethanol at a decreasing concentration; performing a step in 96% ethanol at about 40°C, and a step in 5% ethanol at about 50°C.

The alcoholic extracts obtained as described were gathered in a 50:50 ratio and subjected to decantation for 72 hours, and then, after having spilled the first supernatant, to centrifugation for a time of 1-5 minutes at a rotation rate equal to 3500 rpm; the remaining supernatant was recovered and, gathered with the first one, it was concentrated by ethanol evaporation with use of a thin film distillation system according to the standard protocol, providing the feeding of the extract to be concentrated at a rate of about 500 l/h; operation was by setting a residual vacuum of 06-0.8 bars and the fluid heating the evaporation walls was set at 140°C, thereby obtaining, after ethanol elimination, a concentrated aqueous extract.

The aqueous extract thus obtained was filtered on panel filters with a cutoff of 25 micrometers.

The filtrate thus obtained (fraction C), which comprised harpagoside at a concentration in weight not higher than 1.3%, was collected and used in the mucoadhesion assays.

Fraction D was obtained by subjecting the water-soluble fraction obtained in c. to a step on an adsorbent, high-porosity resin of styrene and DVB copolymer and collecting the adsorbed fraction on resin.

Fractions E and F were obtained by collecting, respectively, the retentate and the eluate derived from the step of the water-soluble fraction obtained in c. on a filter with a cutoff of 1000 Da.

Fractions G and H were obtained by collecting, respectively, the retentate and the eluate derived from the step of the water-soluble fraction obtained in c. on a filter with a cutoff of 2500 Da.

**Example 2**

**Assessment of mucoadhesive ability of extract fractions of the invention on mucin disposed on an inclined plane and comparative assay.**

Measurements were performed by means of an equipment consisting of a 45°-inclined plane, thermostated to 37°C; below said plane, a microbalance was set which performed measurements at regular 1-s intervals and printed recorded measurements on paper. Performed measurements were transcribed and reprocessed on Excel spreadsheet.

The inclined plane acts as support for the biological substrate, consisting of a mucin film. The mucin film was set up by depositing, on the plane consisting of plexiglas kept horizontal, a volume equal to 3ml of a suspension of porcine gastric mucin at an 8% concentration (w/w) in a phosphate buffer, pH 6.4.

The dispersion was brought to dryness at the temperature of 44°C, so as to obtain a film of known surface area equal to 33.6 cm$^2$. Amount, concentration and deposition plane were defined during process development.

An exactly weighed amount of the fraction of the invention and of the reference lyophilized extract was dissolved in solvent (20% EtOH). The samples thus obtained were deposited on the measurement plane with a multichannel pipette at constant time and rate during 6 seconds.

The multichannel pipette was adjusted so as to meter 280 microliters of solution (or dispersion) over the work plane. The total amount measured at each assay is of 1.12 ml per sample (the weight of said volume is measured before the assay and depends on the density on the density of the sample under analysis).

The sample, which would fall on the microbalance, was loaded in the top part of the inclined plane, containing the mucin film, and let slide thereon.

The amount of fallen sample was measured by recording the weight variation as a function of time.

The assay lasted 40 seconds, however it is stressed that after 20 seconds there was no weighing variation.

Measurements in the absence of the mucin film were also performed (measurements indicated as blank) with the same amount of sample and under the same testing conditions, but without the mucin layer deposited on the plane.

Thus, it is possible to assess the intrinsic sliding properties of the sample, regardless of its ability to interact with the biological substrate.

The adhered % was calculated: defined as the amount of sample remained adhered to the mucin film or to the inclined plane (without mucin, blank) at the end of the measurement, normalized with respect to the amount deposited on the inclined plane.

Moreover, it was determined the percentage difference between sample amount adhered to the plane with mucin and sample amount adhered to the plane without mucin. The calculation was performed in accordance with the following equation:

$$\% \text{ difference} = (\text{adhered mucin } \% - \text{adhered blank } \%)/\text{adhered } \% \text{ of the blank.}$$

Where:

- adhered mucin % = sample percentage remained adhered to the mucin film at the end of the measurement
- adhered blank % = sample percentage at the inclined plane at the end of the blank measurements.

<u>Analyzed samples:</u>

The measurements were performed for the following samples:

- Solvent: used as negative control, i.e. as a solution without mucoadhesion, prepared as in the analyzed samples;
- 0.7% sodium alginate in water: used as positive control, i.e. as a substance with known mucoadhesive properties;
- Devil's claw sample: lyophilized fraction C, 20mg/ml in 20% EtOH.
- Comparative devil's claw sample: reference lyophilized sample solubilized in 70% EtOH and diluted in water prior to performing the assay at a concentration of 5 mg/ml.

Obtained data, summarized in Table 1, also demonstrate that despite the elimination of resins (eliminated together with iridoid glycosides) the fraction of devil's claw according to the present invention keeps an excellent mucoadhesive ability.

**Example 3**

**Quantitative HPLC determination of harpagoside**

Preparation of analysis solution.

Accurately weigh 0.50g $\pm$0.025g of ground sample. Extract sample for 30min with 25ml of 80% MeOH by ultrasounds at the temperature of 35°C $\pm$3°C. Centrifuge and decant the extract in a 50ml volumetric flask. Extraction is repeated on the residue, under the same conditions. Centrifuge and decant the extract in the same 50ml volumetric flask. At room temperature, bring gathered extract to a volume of 50ml with the extraction solvent. Filter with a 0.45micrometer cellulose acetate filter and inject in HPLC system.

Chromatographic conditions:

Chromatographic column: C8, 150x4.6mm 5$\mu$ + C8 pre-column, 5$\mu$, 4x3mm column temperature: 20°C
detector: UV-Vis $\lambda$= 278nm
flow: 1ml/min
injection volume: 20ul

The mobile phase consists of solvents: ultrapure water (A) and methanol (B). At the start of the chromatographic analysis the mobile phase consists of 50% water (A) and 50% methanol (B), then, in 15 minutes methanol percent goes to 70% and water percent reduces to 30%. The elution gradient is linear.

| Elution gradient: min. | A % | B % |
| --- | --- | --- |
| 0 | 50 | 50 |
| 15 | 30 | 70 |

Standard harpagoside is solubilized in 80% methanol.

## Claims

1. A fraction of a devil's claw hydroalcoholic extract wherein the iridoid glycoside harpagoside has a concentration expressed as weight percent of ≤1.3% and a concentration of molecules with a molecular weight of > 25000 Da of ≥4%.

2. The fraction according to claim 1, **characterized by** a mucoadhesion higher than 70%, or even higher than 80%, when measured by mucoadhesion assay on an inclined plane.

3. The fraction according to claim 1 or 2 for use in a treatment for the protection of skin or mucous membranes.

4. The fraction for use according to claim 3 wherein said mucous membrane is the oral mucosa, gastric mucosa, intestinal mucosa, the nasal mucosa, the vaginal mucosa, the uterine mucosa, the rectal mucosa.

5. A process for the preparation of a fraction of a devil's claw extract comprising the following steps:

    a. preparing a hydroalcoholic extract of devil's claw roots
    b. obtaining from the extract prepared in a. a concentrated aqueous fraction;
    c. decanting and/or centrifuging and filtering said aqueous fraction, collecting a water-soluble fraction C **characterised by** a concentration of harpagoside, expressed as weight percent, of ≤1.3%, by a concentration of molecules with a molecular weight of > 25000 Da of ≥4% and by a mucoadhesion higher than 70% or even higher than 80% when measured by mucoadhesion assay on an inclined plane,

    wherein said hydroalcoholic extract of step a. is prepared by subjecting devil's claw roots, optionally fragmented, to two or more steps of extraction in ethanol at a decreasing concentration and wherein said ethanol decreasing concentration in step a. is from about 96% ethanol to about 5% ethanol.

6. The process according to claim 5 wherein step a is carried out by performing first step with about 96% ethanol, a step with ethanol at a volume comprised between about 15% and 5% to concentration starting from the residue obtained in the preceding step, then mixing the two extracts 1:1 to have an extract with a resulting ethanol volume comprised between about 45-50% at temperatures which do not exceed 50°

7. The process according to claim 6 wherein the step in 96% ethanol is performed at about 40°C and the next step in 15-5% ethanol is performed at temperatures which do not exceed 50°C.

8. The process according to any one of claims from 5 to 7 wherein said two or more steps in a. comprise a step in about 96% ethanol and a step in about 5% ethanol.

9. A pharmaceutical carrier comprising the fraction according to any one of claims 1 to 4 wherein said carrier promotes the adhesion of active principle to mucous membranes.

10. A composition comprising the fraction according to any one of claims 1 to 4 or the carrier according to claim 9.

11. The composition according to claim 10 for use in a treatment for the protection of skin or mucous membranes.

12. The composition according to claim 11 wherein said mucous membrane are the oral mucosa, gastric mucosa, intestinal mucosa, the nasal mucosa, the vaginal mucosa, the uterine mucosa, the rectal mucosa.

13. The composition according to any one of claims 10 to 12 further comprising substances of natural and/or plant origin having emollient, digestive, prokinetics, cholagogue, carminative, prebiotic, relaxing, excipient, preservative, humectant properties.

14. The composition according to claim 13 wherein said substances of natural and/or plant origin are selected from one or more from: gentian root extract, boldo leaves extract, milk thistle fruits extract, artichoke leaves extract, taraxacum root extract, anise (or aniseed) fruit extract, rosemary leaves extract, mint leaves extract, marjoram leaves extract, cumin fruit extract, coriander fruit extract, ginger root extract, fennel fruit extract, caraway fruit extract, althea root extract, aloe vera gel, arnica flower extract, charcoal, inulin, jojoba oil.

15. The composition according to any one of claims 10 to 14 in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

16. The composition according to any one of claims 10 to 15 wherein said composition is a pharmaceutical composition, is comprised in or consists of a medical device, is comprised in or consists of a food supplement or is comprised in a medical food.

**Patentansprüche**

1. Fraktion eines wässrig-alkoholischen Teufelskrallenextrakts, wobei das Iridoidglykosid Harpagosid über eine Konzentration verfügt, die als Gewichtsprozent von $\leq 1,3 \%$ ausgedrückt wird, und über eine Konzentration von Molekülen mit einem Molekulargewicht von > 25000 Da von $\geq 4 \%$ verfügt.

2. Fraktion gemäß Anspruch 1, **gekennzeichnet durch** eine Mucoadhäsion, die höher als 70 % oder sogar höher als 80 % ist, wenn sie durch ein Mucoadhäsionsassay auf einer schiefen Ebene gemessen wird.

3. Fraktion gemäß Anspruch 1 oder 2 zur Verwendung bei einer Behandlung zum Schutz von Haut oder Schleimhäuten.

4. Fraktion zur Verwendung gemäß Anspruch 3, wobei die Schleimhaut die Mundschleimhaut, die Magenschleimhaut, die Darmschleimhaut, die Nasenschleimhaut, die Vaginalschleimhaut, die Gebärmutterschleimhaut, die Rektalschleimhaut ist.

5. Verfahren zur Herstellung einer Fraktion eines Teufelskrallenextrakts, das die folgenden Schritte umfasst:

   a) Herstellen eines wässrig-alkoholischen Extrakts aus Teufelskrallenwurzeln;
   b) Erhalten aus dem in a) hergestellten Extrakt einer konzentrierten wässrigen Fraktion;
   c) Dekantieren und/oder Zentrifugieren und Filtern der wässrigen Fraktion, wobei eine wasserlösliche Fraktion C gewonnen wird, **gekennzeichnet durch** eine Harpagosid-Konzentration, die als Gewichtsprozent, von $\leq 1,3 \%$, ausgedrückt wird, durch eine Konzentration von Molekülen mit einem Molekulargewicht von > 25000 Da von $\geq 4 \%$ und durch eine Mucoadhäsion, die höher als 70 % und sogar höher als 80 % ist, wenn sie durch eine Mucoadhäsionsassay auf einer schiefen Ebene gemessen wird,

   wobei der wässrig-alkoholische Extrakt von Schritt a) durch Aussetzen der Teufelskrallenwurzeln, optional fragmentiert, zweier oder mehrerer Schritte einer Extraktion in Ethanol bei einer abnehmenden Konzentration hergestellt wird und wobei die Konzentrationsabnahme von Ethanol in Schritt a) von ungefähr 96 % Ethanol bis ungefähr 5 % Ethanol verläuft.

6. Verfahren gemäß Anspruch 5, wobei der Schritt a folgendermaßen durchgeführt wird: durch einen ersten Schritt mit ungefähr 96 % Ethanol, einen Schritt mit Ethanol bei einem Volumen zwischen ungefähr 15 % und 5 % Konzentration, beginnend bei dem in dem vorangehenden Schritt erhaltenen Rückstand, dann Mischen der zwei Extrakte im Verhältnis 1:1, um einen Extrakt mit einem resultierenden Ethanolvolumen zwischen ungefähr 45 und 50 % bei Temperaturen unterhalb von 50° zu erhalten.

7. Verfahren gemäß Anspruch 6, wobei der Schritt in 96 % Ethanol bei ungefähr 40°C und der nächste Schritt in 15 - 5 % Ethanol bei Temperaturen unterhalb von 50°C durchgeführt wird.

8. Verfahren gemäß einem der Ansprüche 5 bis 7, wobei die zwei oder mehr Schritte in a) einen Schritt in ungefähr 96 % Ethanol und einen Schritt in ungefähr 5 % Ethanol umfassen.

9. Pharmazeutischer Träger, der umfasst: die Fraktion gemäß einem der Ansprüche 1 bis 4, wobei der Träger die Adhäsion von Wirkstoffen an den Schleimhäuten fördert.

10. Zusammensetzung, die umfasst: die Fraktion gemäß einem der Ansprüche 1 bis 4 oder den Träger gemäß Anspruch 9.

**11.** Zusammensetzung gemäß Anspruch 10 zur Verwendung in einer Behandlung zum Schutz von Haut und Schleimhäuten.

**12.** Zusammensetzung gemäß Anspruch 11, wobei die Schleimhaut die Mundschleimhaut, die Magenschleimhaut, die Darmschleimhaut, die Nasenschleimhaut, die Vaginalschleimhaut, die Gebärmutterschleimhaut, die Rektalschleimhaut ist.

**13.** Zusammensetzung gemäß einem der Ansprüche 10 bis 12, die weiterhin umfasst: Substanzen natürlichen und/oder pflanzlichen Ursprungs, die über emolliente, digestive, Prokinetic-, galletreibende, karminative, praebiotische, entspannende, Hilfsstoff-, konservierende, Befeuchtungsmittel-Eigenschaften verfügen.

**14.** Zusammensetzung gemäß Anspruch 13, wobei die Substanzen natürlichen und/oder pflanzlichen Ursprungs umfassen: Enzianwurzelextrakt, Boldoblätterextrakt, Mariendistelfrüchteextrakt, Artischockenblätterextrakt, Löwenzahnwurzelextrakt, Anis (oder Anissamen)-Fruchtextrakt, Rosmarinblätterextrakt, Minzblätterextrakt, Majoranblätterextrakt, Kreuzkümmelfruchtextrakt, Korianderfruchtextrakt, Ingwerwurzelextrakt, Fenchelfruchtextrakt, Kümmelfruchtextrakt, Eibischwurzelextrakt, Aloe-Vera-Gel, Arnikablumenextrakt, Kohle, Inulin und/oder Jojobaöl.

**15.** Zusammensetzung gemäß einem der Ansprüche 10 bis 14 in der Form einer Kapsel, einer Tablette, einer Lutschtablette, eines Granulats, eines Pulvers, eines Sirups, eines Elixiers, einer Hartgelatine, einer Weichgelatine, einer Suspension, einer Emulsion, einer Lösung, eines Zäpfchens, einer Creme, eines Gels, eines Sprays, einer Salbe, einer Pomade, einer Paste, einer Öl-in-Wasser-Emulsion, einer Öl-in-Gel-Emulsion, einer Gel-in-Öl-Emulsion.

**16.** Zusammensetzung gemäß einem der Ansprüche 10 bis 15, wobei die Zusammensetzung eine pharmazeutische Zusammensetzung ist, aus einem Medizinprodukt besteht oder darin enthalten ist, aus einem Nahrungsergänzungsmittel besteht oder darin enthalten ist, oder in einem medizinischen Nahrungsmittel enthalten ist.


**Revendications**

**1.** Fraction d'un extrait hydro-alcoolique de griffe du diable dans laquelle le glycoside d'iridoïde harpagoside a une concentration exprimée en pourcentage en poids ≤1,3 % et une concentration de molécules ayant un poids moléculaire >25 000 Da ≥4 %.

**2.** Fraction selon la revendication 1, **caractérisée par** une muco-adhérence, mesurée par un test de muco-adhérence sur plan incliné, supérieure à 70 %, voire supérieure à 80 %.

**3.** Fraction selon la revendication 1 ou 2 destinée à être utilisée dans la protection de la peau ou des membranes muqueuses.

**4.** Fraction pour son utilisation selon la revendication 3 dans laquelle ladite membrane muqueuse est la membrane buccale, la muqueuse gastrique, la muqueuse intestinale, la muqueuse nasale, la muqueuse vaginale, la muqueuse utérine, la muqueuse rectale.

**5.** Procédé de préparation d'une fraction d'un extrait de griffe du diable comprenant les étapes suivantes :

a. préparation d'un extrait hydro-alcoolique de racines de griffe du diable
b. obtention à partir de l'extrait préparé en a. d'une fraction aqueuse concentrée ;
c. décantation et/ou centrifugation et filtration de ladite fraction aqueuse, collecte d'une fraction C hydrosoluble **caractérisée par** une concentration d'harpagoside, exprimée en pourcentage en poids, ≤1,3 %, par une concentration de molécules ayant un poids moléculaire >25 000 Da ≥4 % et par une muco-adhérence, mesurée par un test de muco-adhérence sur plan incliné, supérieure à 70 %, voire supérieure à 80 %,

dans lequel ledit extrait hydro-alcoolique de l'étape a. est préparé par soumission de racines de griffe du diable, éventuellement fragmentées, à deux étapes d'extraction ou plus dans de l'éthanol à une concentration décroissante et dans lequel ladite concentration décroissante d'éthanol à l'étape a. est d'environ 96 % d'éthanol à environ 5 % d'éthanol.

**6.** Procédé selon la revendication 5 dans lequel l'étape a. est réalisée par mise en oeuvre d'une première étape avec

EP 2 845 624 B1

environ 96 % d'éthanol, d'une étape avec de l'éthanol à un volume compris entre environ 15 et 5 % jusqu'à une concentration démarrant du résidu obtenu à l'étape précédente, puis mélange des deux extraits 1:1 pour obtenir un extrait ayant un volume d'éthanol total compris entre environ 45 et 50 % à des températures n'excédant pas 50 °C.

7. Procédé selon la revendication 6 dans lequel l'étape dans 96 % d'éthanol est mise en oeuvre à environ 40 °C et l'étape suivante dans 15 à 5 % d'éthanol est mise en oeuvre à des températures n'excédant pas 50 °C.

8. Procédé selon l'une quelconque des revendications 5 à 7 dans lequel lesdites deux étapes ou plus en a. comprennent une étape dans environ 96 % d'éthanol et une étape dans environ 5 % d'éthanol.

9. Véhicule pharmaceutique comprenant la fraction selon l'une quelconque des revendications 1 à 4 dans lequel ledit véhicule favorise l'adhérence du principe actif aux membranes muqueuses.

10. Composition comprenant la fraction selon l'une quelconque des revendications 1 à 4 ou le véhicule selon la revendication 9.

11. Composition selon la revendication 10 destinée à être utilisée dans la protection de la peau ou des membranes muqueuses.

12. Composition selon la revendication 11 dans laquelle ladite membrane muqueuse est la membrane buccale, la muqueuse gastrique, la muqueuse intestinale, la muqueuse nasale, la muqueuse vaginale, la muqueuse utérine, la muqueuse rectale.

13. Composition selon l'une quelconque des revendications 10 à 12 comprenant en outre des substances d'origine naturelle et/ou végétale ayant des propriétés émollientes, digestives, procinétiques, cholagogues, carminatives, prébiotiques, relaxantes, excipientes, conservatrices, hydratantes.

14. Composition selon la revendication 13 dans laquelle lesdites substances d'origine naturelle et/ou végétale sont choisies parmi une ou plusieurs des suivantes : extrait de racine de gentiane, extrait de feuilles de boldo, extrait de fruits de chardon-Marie, extrait de feuilles d'artichaut, extrait de racines de pissenlit, extrait de fruits d'anis, extrait de feuilles de romarin, extrait de feuilles de menthe, extrait de feuilles de marjolaine, extrait de fruits de cumin, extrait de fruits de coriandre, extrait de racine de gingembre, extrait de fruits de fenouil, extrait de fruits de carvi, extrait de racine d'althéa, gel d'Aloe vera, extrait de fleurs d'arnica, charbon, inuline, huile de jojoba.

15. Composition selon l'une quelconque des revendications 10 à 14 sous la forme d'une capsule, d'un comprimé, d'une pastille à sucer, d'un granulé, d'une poudre, d'un sirop, d'un élixir, d'une gélatine dure, d'une gélatine molle, d'une suspension, d'une émulsion, d'une solution, d'un suppositoire, d'une crème, d'un gel, d'un spray, d'une pommade, d'une pâte, d'une émulsion huile dans l'eau, d'une émulsion eau dans l'huile, d'une émulsion huile dans gel, d'une émulsion gel dans l'huile.

16. Composition selon l'une quelconque des revendications 10 à 15 dans laquelle ladite composition est une composition pharmaceutique, est contenue dans ou consiste en un dispositif médical, est contenue dans ou consiste en un supplément alimentaire ou est contenue dans un alicament.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J.D. SMART.** *Advanced drug delivery reviews,* 2005, vol. 57, 1556-1568